# NEUE EUROPÄISCHE PATENTSCHRIFT

(11) **EP 1 596 954 B2**
(45) Veröffentlichungstag und Bekanntmachung des Hinweises auf die Entscheidung über den Einspruch: **10.12.2014**
(45) Hinweis auf die Patenterteilung: 26.05.2010
(21) Anmeldenummer: 03812597.7
(22) Anmeldetag: 11.12.2003
(51) Int. Cl.: B01D 3/14, B01D 3/40, C07C 7/08

(54) **VERFAHREN ZUR EXTRAKTIVDESTILLATION**
METHOD FOR EXTRACTIVE DISTILLATION
PROCEDE DE DISTILLATION EXTRACTIVE

(30) Priorität: 12.12.2002 DE 10258160
(43) Veröffentlichungstag der Anmeldung: 23.11.2005
(73) Patentinhaber: BASF SE, 67056 Ludwigshafen (DE)
(72) Erfinder: HEIDA, Bernd, 67158 Ellerstadt (DE)
(86) Internationale Anmeldenummer: PCT/EP2003/014074
(87) Internationale Veröffentlichungsnummer: WO 2004/052492

(56) Entgegenhaltungen:
- EP-A- 0 755 707
- EP-A1- 1 317 947
- WO-A-2004/011406
- DE-A- 10 002 806
- DE-A- 10 105 660
- DE-A- 10 233 620
- US-B1- 6 550 274

## Beschreibung

Die Erfindung betrifft ein Verfahren zur Auftrennung eines Ausgangsgemisches aus zwei oder mehreren Komponenten durch Extraktivdestillation in einer Trennwandkolonne.

Die Extraktivdestillation ist ein bekanntes Verfahren zur destillativen Auftrennung von Gemischen aus Komponenten, die sich nur geringfügig in ihren relativen Flüchtigkeiten unterscheiden oder die azeotrop sieden. Die Extraktivdestillation wird unter Zugabe eines selektiven Lösungsmittels, auch als Extraktionsmittel bezeichnet, durchgeführt, das gegenüber dem aufzutrennenden Gemisch wesentlich höher siedet und das durch seine selektive Affinität zu einzelnen Komponenten des aufzutrennenden Gemisches die Unterschiede in den relativen Flüchtigkeiten derselben erhöht. Ein wesentliches Kriterium für die Auswahl des geeigneten selektiven Lösungsmittels ist es, denjenigen Aufnehmer herauszufinden, der die Trennung mit einem Minimum an zirkulierender Aufnehmerphase ermöglicht, der sich also durch eine ausreichend hohe Aufnahmekapazität kennzeichnet.

Es ist bekannt, für komplexere Trennaufgaben, in der Regel für Gemische von mindestens drei Komponenten, wobei die einzelnen Komponenten jeweils in reiner Form erhalten werden sollen, Trennwandkolonnen einzusetzen. Sie weisen eine Trennwand auf, das heißt ein in der Regel in Kolonnenlängsrichtung ausgerichtetes ebenes Blech, das eine Quervermischung der Flüssigkeits- und Brüdenströmen in Teilbereichen der Kolonne unterbindet. Trennwandkolonnen sind gegenüber klassischen Destillationskolonnen wirtschaftlich vorteilhaft, da sie Trennaufgaben, für die normaler Weise zwei Kolonnen notwendig sind, in einem einzigen Apparat bewältigen, wobei die Investitions- und Energiekosten deutlich niedriger liegen.

Es ist bekannt, Extraktivdestillationen in Trennwandkolonnen durchzuführen.

Ein derartiges Verfahren ist beispielsweise in DE-C 199 58 464 beschrieben. Durch eine besondere konstruktive Ausgestaltung der Trennwandkolonne, und zwar indem der Entnahmebereich am oberen Ende der Trennwand geschlossen wird, kann die Temperatur in diesem Kolonnenbereich durch Steuerung des darin herrschenden Drucks geregelt werden. Da der Druck im oberseitig geschlossenen Entnahmebereich gegenüber dem Betriebsdruck in der Kolonne verändert werden kann, können die Druckdifferenzen zur Steuerung der in die durch die Trennwand aufgeteilten Teilbereiche der Kolonne eintretenden Dampfströme genutzt werden.

Aus der nicht vorveröffentlichten europäischen Patentanmeldung EP-A 1 317 947 ist ein Verfahren zur Destillation eines Gemisches mit drei oder mehreren Komponenten in einer Kolonne mit mindestens drei Destillationszonen bekannt, die als Trennwandkolonne mit bis zum oberen Kolonnenende durchgezogener Trennwand, die den Kolonneninnenraum in einen ersten oberen Kolonnenbereich, einen zweiten oberen Kolonnenbereich sowie einen unteren gemeinsamen Kolonnenbereich aufteilt, ausgebildet sein kann, mit Zuführung des Ausgangsgemisches und des selektiven Lösungsmittels in den ersten oberen Kolonnenbereich und Abzug von Produktströmen jeweils am oberen Ende des ersten oberen Kolonnenbereichs, des zweiten oberen Kolonnenbereichs sowie des unteren gemeinsamen Kolonnenbereiches.

Aus der nicht vorveröffentlichten Patentanmeldung WO-A-04011406 ist ein kontinuierliches Verfahren zur Auftrennung eines C₄-Schnittes durch Extraktivdestillation mit einem selektiven Lösungsmittel bekannt, wobei eine Extraktivdestillationskolonne eingesetzt wird mit in Längsrichtung derselben angeordneter Trennwand, die bis zum oberen Ende der Extraktivdestillationskolonne durchgezogen ist, unter Ausbildung eines ersten oberen Teilbereichs, eines zweiten oberen Teilbereichs und eines unteren gemeinsamen Kolonnenbereichs und wobei aus dem ersten oberen Teilbereich ein die Butane umfassender Kopfstrom, aus dem zweiten oberen Teilbereich ein die Butene umfassender Kopfstrom und aus dem unteren gemeinsamen Kolonnenbereich ein die Kohlenwasserstoffe aus dem C₄-Schnitt umfassender Strom abgetrennt wird, die im selektiven Lösungsmittel besser löslich sind als die Butane und die Betene.

Es war demgegenüber Aufgabe der Erfindung, ein wirtschaftlicheres, insbesondere bezüglich des Energie- und Lösungsmittelverbrauches günstigeres Verfahren zur Verfügung zu stellen.

Die Aufgabe wird durch ein Verfahren wie Anspruch 1.

Als Ausgangsgemische werden in der Regel Gemische von Kohlenwasserstoffen oder von anderen organischen Komponenten in Frage kommen, die sich aufgrund der geringen Unterschiede in den Flüchtigkeiten mindestens zweier Komponenten oder aufgrund der Ausbildung von Azeotropen nur durch Zugabe eines selektiven Lösungsmittels, das die relativen Flüchtigkeiten verändert, auftrennen lassen.

Das Ausgangsgemisch wird dem ersten Teilbereich der Trennwandkolonne, häufig etwa im mittleren Drittel desselben, aufgegeben. Je nach konkreter Zusammensetzung des aufzutrennenden Ausgangsgemisches kann es möglich sein, dass die Komponente oder das Komponentengemisch mit der größten Flüchtigkeit von den übrigen Komponenten des Ausgangsgemisches destillativ einfach abgetrennt werden kann. In diesem Fall ist keine Zugabe von selektivem Lösungsmittel im oberen Bereich des ersten Teilsbereichs erforderlich, um einen Kopfstrom aus einer einzigen, reinen Komponenten, mit der höchsten Flüchtigkeit oder eines Bündels von Komponenten mit der höchsten Flüchtigkeit abzutrennen.

In der Regel ist jedoch die Abtrennung einer reinen Komponente oder eines Bündels an Komponenten mit der höchsten Flüchtigkeit im ersten Teilbereich der Trennwandkolonne nur unter Zugabe eines selektiven Lösungsmittels im oberen Bereich des ersten Teilbereichs und somit durch Gegenstromführung mit dem aufzutrennenden Ausgangsgemisch möglich. In diesem Fall wird das selektive Lösungsmittel bei geeigneten thermodynamischen Bedingungen, in der Regel bei möglichst niedrigen Temperaturen aufgegeben, und belädt sich mit den Komponenten aus dem Ausgangsgemisch, zu denen es eine höhere Affinität hat, wogegen die Komponente oder die Komponenten, zu denen es eine niedrigere Affinität hat, das heißt die im selektiven Lösungsmittel schlecht löslichen Komponenten, in der Dampfphase verbleiben und als Kopfstrom abgezogen werden.

Das mit den Komponenten aus dem Ausgangsgemisch beladene Lösungsmittel, zu dem dieses eine größere Affinität hat als zu der oder den Komponenten, die als Kopfstrom aus dem ersten Teilbereich abgetrennt wurden, strömt am unteren Ende der Trennwand dampfförmig in den zweiten Teilbereich der Trennwandkolonne ein.

Im zweiten Teilbereich der Trennwandkolonne kann, je nach konkreter Zusammensetzung des aufzutrennenden Ausgangsgemisches, eine einfache destillative Auftrennung der im selektiven Lösungsmittel besser löslichen Komponenten möglich sein, es kann jedoch auch sein, dass die Auftrennung desselben nur unter Zugabe von selektivem Lösungsmittel im Gegenstrom auf den oberen Bereich des zweiten Teilbereichs, das heißt durch Extraktivdestillation, möglich ist.

Der aus dem zweiten Teilbereich abgezogene Kopfstrom kann wiederum, wie im Falle des aus dem ersten Teilbereich abgezogenen Kopfstroms, eine einzige, reine Komponente oder ein Bündel von Komponenten, mit einem bestimmten Siedebereich umfassen.

Die Kopfströme aus dem ersten wie auch aus dem zweiten Teilbereich werden, wie üblich, in Kondensatoren am Kolonnenkopf kondensiert, teilweise als Rücklauf wieder auf die Kolonne aufgegeben und im übrigen abgezogen.

Für die Kopfströme werden in der Regel bestimmte Reinheitsanforderungen, das heißt Spezifikationen, vorgegeben.

Da sich das jeweils aufzutrennende Gemisch in den beiden Teilbereichen der Trennwandkolonne hinsichtlich Menge und Zusammensetzung stets voneinander unterscheidet, wird, sofern die Auftrennung durch Extraktivdestillation erfolgt, eine jeweils bestimmte optimale Menge an selektivem Lösungsmittel erforderlich sein, um die vorgegebenen Spezifikationen in den Kopfströmen zur erreichen.

Nach dem erfindungsgemäßen Verfahren zur Extraktivdestillation in einer Kolonne mit einer bis zum oberen Ende durchgezogenen Trennwand ist es in einfacher Weise möglich, in einem Teilbereich die für die jeweilige Trennaufgabe optimale Menge an selektivem Lösungsmittel aufzugeben.

Aus dem Kolonnensumpf wird Lösungsmittel abgezogen, das noch mit einer oder mehreren Komponenten beladen ist, die im selektiven Lösungsmittel am besten löslich sind. Das beladene Lösungsmittel wird anschließend in einer Ausgaserkolonne unter geeigneten thermodynamischen Bedingungen von den darin gelösten Komponenten befreit und das gereinigte Lösungsmittel in der Regel in die Extraktivdestillationskolonne rezykliert.

Aus dem unteren gemeinsamen Kolonnenbereich wird ein dampfförmiger Strom abgezogen, bevorzugt teilweise oder vollständig kondensiert und vollständig oder teilweise abgezogen und im übrigen wieder als Rücklauf in die Extraktivdestillationskolonne zurückgeführt. Im Kolonnenteil unterhalb dieser Entnahmestelle werden die gelösten Komponenten vollständig aus dem Lösungsmittel ausgegast.

Es ist darüber hinaus möglich, je nach durchzuführender Trennaufgabe aus jedem geeigneten Kolonnenbereich, insbesondere aus dem ersten Teilbereich und/oder dem zweiten Teilbereich und/oder dem unteren gemeinsamen Kolonnenbereich einen oder mehrere zusätzliche Seitenströme abzuziehen.

In einer energetisch besonders günstigen Verfahrensvariante wird von ein oder mehreren thermodynamisch geeigneten Stufen der Extraktivdestillationskolonne jeweils ein Flüssigkeitsstrom abgezogen, durch Wärmeintegration mit dem heißen, entgasten Lösungsmittel teilweise oder vollständig verdampft und derselben Trennstufe oder oberhalb der Trennstufe, von der der Flüssigkeitsstrom abgezogen wurde, in die Extraktivdestillationskolonne zurückgeführt. Dadurch kann der Gesamtenergieverbrauch wesentlich gesenkt werden, typischerweise um etwa 40 bis 60 %.

Je nach Zusammensetzung des der Extraktivdestillationskolonne zugeführten Ausgangsgemisches sowie der vorgegebenen Spezifikationen für die in der Extraktivdestillationskolonne abzutrennenden Fraktionen kann die Länge der Trennwand sowie ihre Positionierung in Bezug auf die Kolonnenachse unterschiedlich ausgestaltet sein. So ist es beispielsweise möglich, die Trennwand mittig oder außermittig anzuordnen. Eine außermittige Anordnung wird häufig vorteilhaft sein, da die Flüssigkeits- und Dampfbelastung in den beiden Teilbereichen in der Regel unterschiedlich ist.

In einer bevorzugten Ausgestaltung sind im ersten und/oder im zweiten Teilbereich der Extraktivdestillationskolonne, jeweils oberhalb der Zuführung des selektiven Lösungsmittels Rückwaschböden für mit dem Dampfstrom mitgerissenes selektives Lösungsmittel vorgesehen, häufig drei bis fünf Böden. Obwohl es bezüglich der einsetzbaren Typen grundsätzlich keine Einschränkungen gibt, sind Böden für kleine Flüssigkeitsbelastungen, insbesondere Ventil-, Glocken- oder Thormannböden besonders geeignet.

Durch den Einsatz von Rückwaschböden können bezüglich des Restgehalts an Lösungsmittel besonders reine Kopffraktionen erhalten werden.

Das wichtigstes Anwendungsgebiet für das erfindungsgemäße Verfahren und der erfindungsgemäßen Extraktivdestillationskolonne ist die Petrochemie, insbesondere die Auftrennung von C₄-Schnitten, C₅-Schnitten, die Auftrennung von Aromatengemischen, insbesondere von Benzol-Toluol-Xylol-Gemischen oder der Xylol-Isomere.

Die Erfindung wird im Folgenden anhand eines Ausführungsbeispiels sowie einer Zeichnung näher erläutert.

Es zeigen im Einzelnen:
- Figur 1: die schematische Darstellung einer ersten Ausführungsform einer Extraktivdestillationskolonne,
- Figur 2: eine Ausführungsform einer eingesetzten Extraktivdestillationskolonne,
- Figur 3: eine andere Ausführungsform einer eingesetzten Extraktivdestillationskolonne,
- Figur 4: eine Ausführungsform mit Rückwaschböden,
- Figur 5: eine weitere Ausführungsform mit integrierter Lösungsmittelausgasung,
- Figur 6: eine Ausführungsform mit Rückwaschböden und integrierter Lösungsmittelausgasung und
- Figur 7: eine Ausführungsform mit Wärmeintegration.

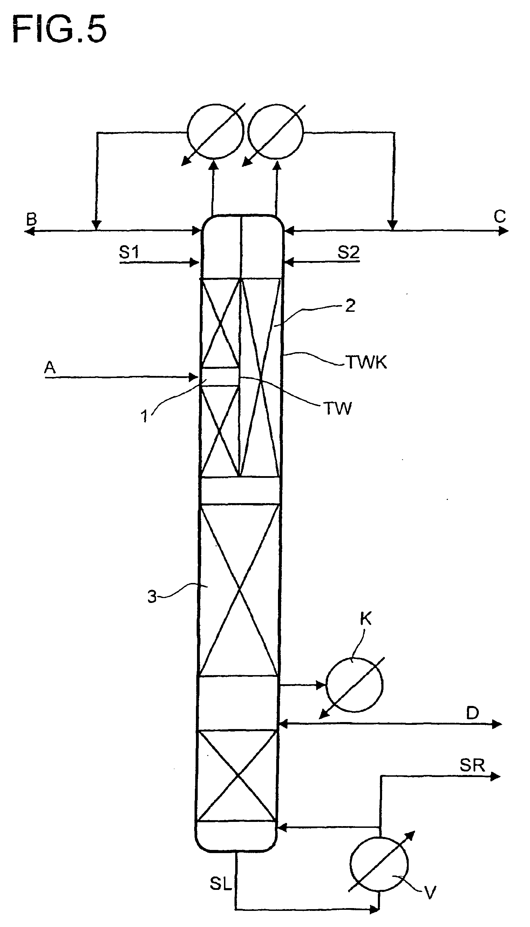

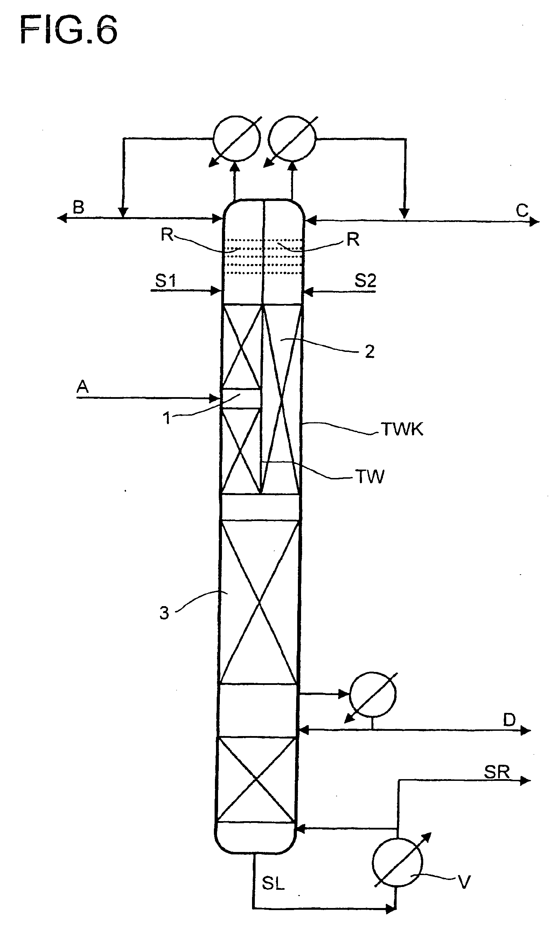

Die Figuren 5 und 6 verdeutlichen erfindungsgemäß einsetzbare Extraktivdestillationskolonnen.

In den Figuren bezeichnen gleiche Bezugszeichen jeweils gleiche oder entsprechende Merkmale.

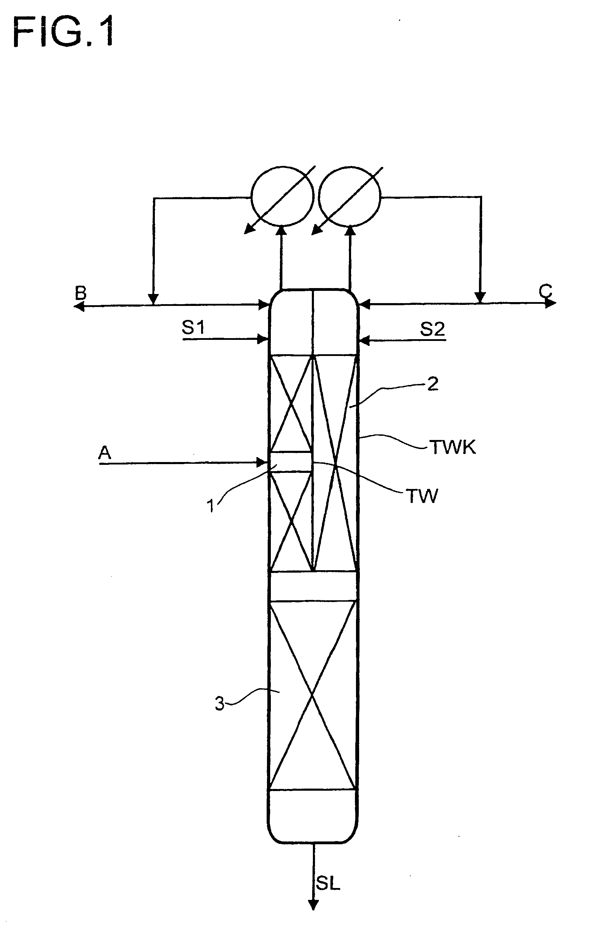

Die in Fig. 1 dargestellte Extraktivdestillationskolonne ist als Trennwandkolonne TKW ausgebildet, mit in Kolonnenlängsrichtung, bis zum oberen Kolonnenende durchgezogener Trennwand TW. Die Trennwand TW teilt den Kolonneninnenraum in einen ersten Teilbereich 1, einen zweiten Teilbereich 2 sowie einen unteren gemeinsamen Kolonnnenbereich 3. Dem ersten Kolonnenbereich 1 wird das aufzutrennende Ausgangsgemisch A, etwa in der Mitte desselben aufgegeben. Ein erster Strom des selektiven Lösungsmittels wird als Strom S1 im oberen Bereich des ersten Teilbereichs 1 und ein zweiter Strom des selektiven Lösungsmittels als Strom S2 dem oberen Bereich des Teilbereichs 2 aufgegeben. Aus dem Teilbereich 1 wird ein erster Kopfstrom B abgezogen, in einem Kondensator am Kolonnenkopf kondensiert, teilweise als Rücklauf wieder auf die Kolonne aufgegeben und im übrigen abgezogen. Analog wird aus dem zweiten Teilbereich 2 ein zweiter Kopfstrom C abgezogen, in einem Kondensator am Kolonnenkopf kondensiert, teilweise als Rücklauf wieder auf den zweiten Teilbereich aufgegeben und im übrigen abgezogen.

Aus dem Kolonnensumpf wird beladenes selektives Lösungsmittel, Strom SL, abgezogen.

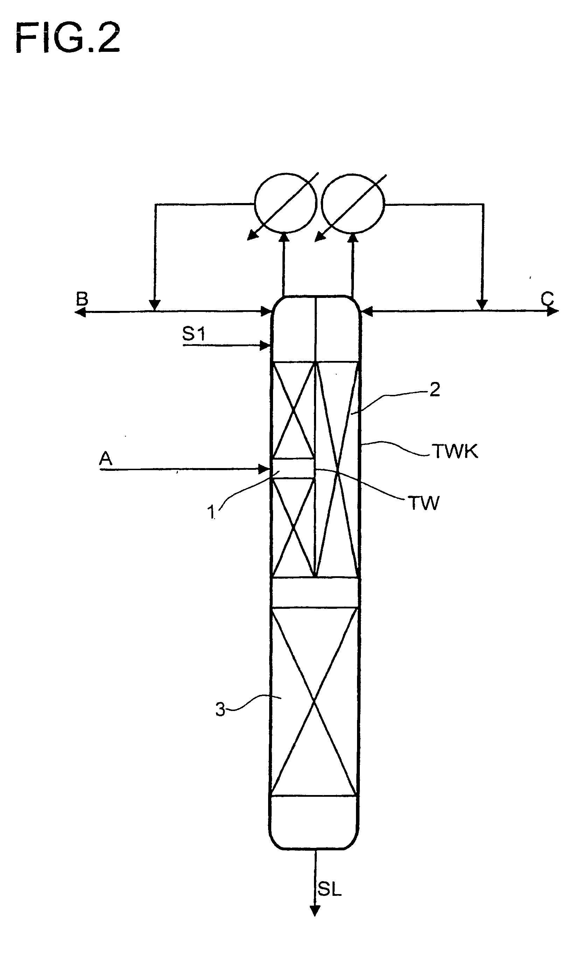

Die in Fig. 2 dargestellte Ausführungsform unterscheidet sich von der Ausführungsform in Fig. 1 dadurch, dass ein einziger Strom von selektivem Lösungsmittel, Strom S1, auf den Teilbereich 1 aufgegeben wird. Die destillative Auftrennung im Teilbereich 2 erfolgt ohne Zugabe von selektivem Lösungsmittel.

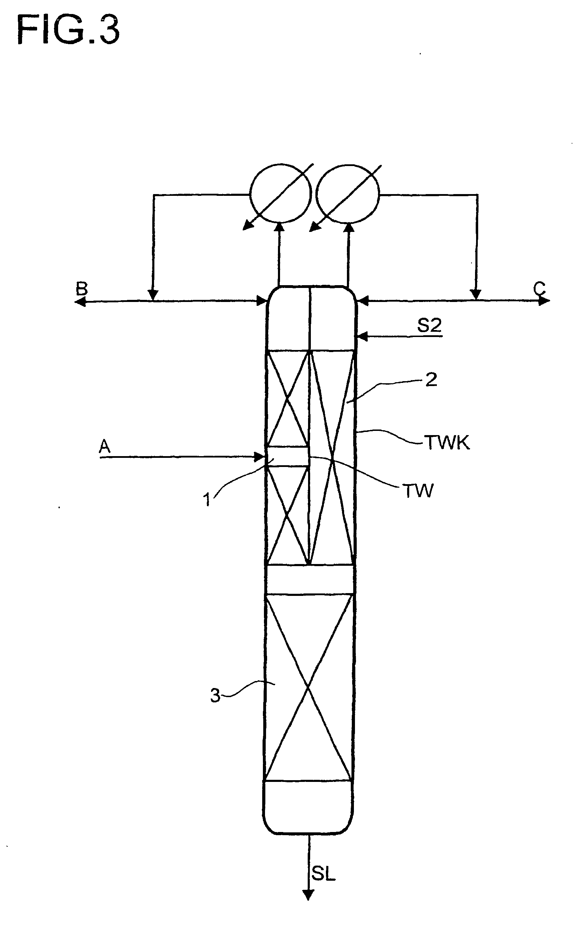

Auch in der Ausführungsform in Fig. 3 wird lediglich ein einziger Strom an selektivem Lösungsmittel aufgegeben, abweichend von der Ausführungsform in Fig. 2, jedoch Strom S2 im oberen Bereich des Teilbereichs 2.

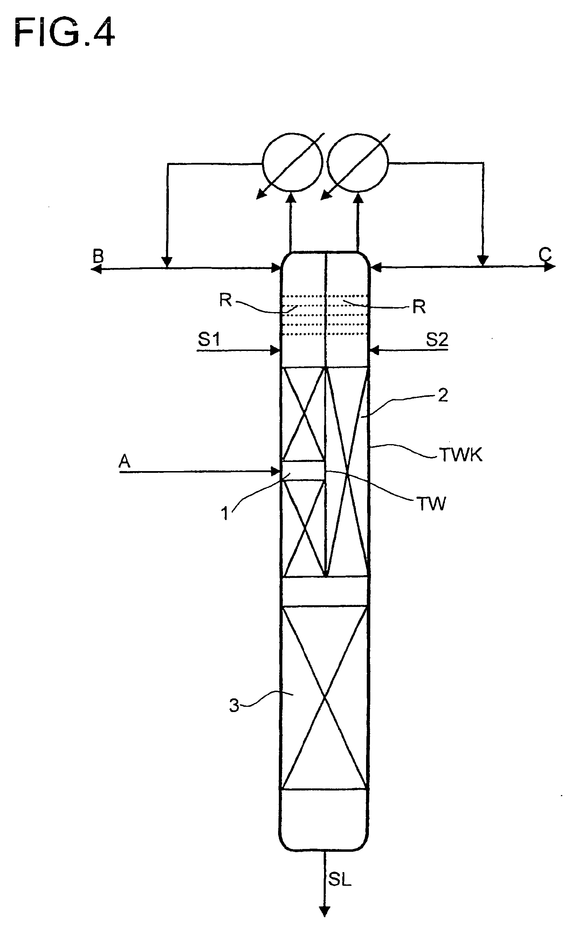

Die Ausführungsform in Fig. 4 enthält zusätzlich zu der in Fig. 1 dargestellten Grundform Rückwaschböden R, die jeweils oberhalb der Zuführung der Lösungsmittelströme S1 und S2 in den Teilbereichen 1 und 2 angeordnet sind.

In der in Fig. 5 dargestellten Ausführungsform wird aus dem unteren gemeinsamen Kolonnenbereich 3 ein dampfförmiger Seitenstrom abgezogen, in einem Kondensator teilweise oder vollständig kondensiert, teilweise als Strom D abgezogen und im übrigen wieder als Rücklauf in die Kolonne zurückgeführt. Unterhalb des Seitenabzugs für Strom D wird das beladene Lösungsmittel durch Wärmezuführung über den Sumpfverdampfer V vollständig ausgegast und als gereinigtes Lösungsmittel, Strom SR, abgezogen. Das gereinigte Lösungsmittel wird bevorzugt, nach Abkühlung in einem Kondensator, in die Extraktivdestillation rezykliert.

Die in Fig. 6 dargestellte Ausführungsform unterscheidet sich von der Darstellung in Fig. 5 durch die Anordnung von Rückwaschböden R in den Teilbereich 1 und 2, jeweils oberhalb der Zuführung der Lösungsmittelströme S1 und S2.

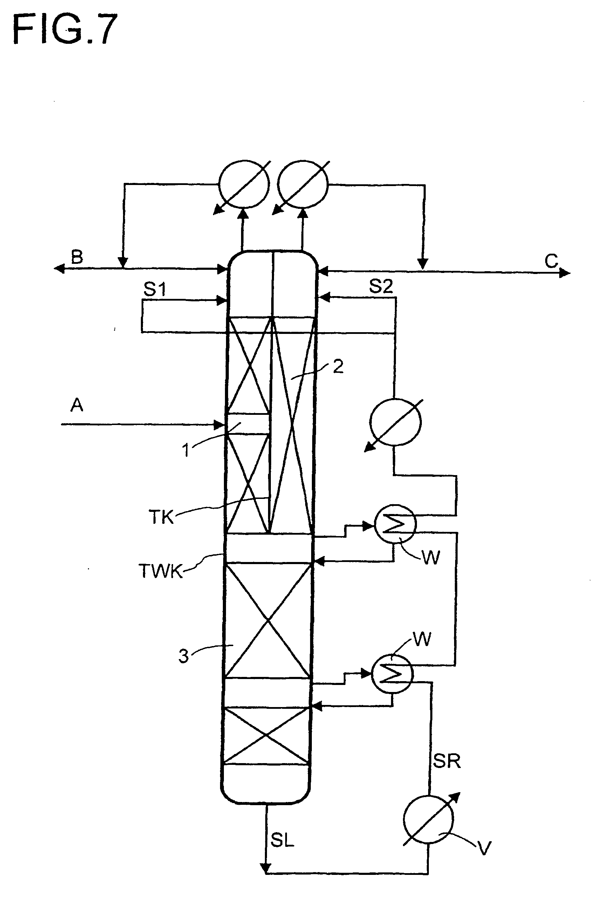

Fig. 7 zeigt eine energetisch besonders günstige Verfahrensvariante, mit Wärmeintegration, wobei heißes, gereinigtes Lösungsmittel, Strom SR, in Wärmetauschern W aus der Extraktivdestillationskolonne abgezogene Flüssigkeit erwärmen.

### Beispiel:

Das Beispiel fällt nicht unter eine Verfahrensfürhrung nach Anspruch 1.

Auftrennung eines C₄-Schnittes durch Extraktivdestillation

### Auftrennung eines C₄-Schnittes durch Extraktivdestillation

Die Trennaufgabe bestand darin, einen C₄-Schnitt mit der nachfolgenden Zusammensetzung in Gew.-%:

| | |
|---|---|
| Propadien | 0,03 |
| Propen | 0,02 |
| Propin | 0,06 |
| n-Butan | 5,74 |
| i-Butan | 2,44 |
| n-Buten | 13,88 |
| i-Buten | 25,63 |
| trans-Buten-2 | 4,44 |
| cis-Buten-2 | 2,95 |
| 1,3-Butadien | 43,81 |
| 1,2-Butadien | 0,14 |
| Butin-1 | 0,13 |
| Vinylacetylen | 0,73 |

in eine Butane-Fraktion mit 90 Gew.-% Butanen und eine Butene-Fraktion mit 98 Gew.-% Butenen, jeweils als Kopfströme sowie einen Sumpfstrom, der neben dem selektiven Lösungsmittel 99 % des 1,3-Butadiens sowie die besser als 1,3-Butadien löslichen Komponenten enthält, aufzutrennen.

Als selektives Lösungsmittel wurde N-Methylpyrrolidon als wässrige Lösung mit 8,3 Gew.-% Wasser eingesetzt.

Es wurde jeweils ein Feedstrom von 31.250 kg/h eines C₄-Schnitts mit der oben angegebenen Zusammensetzung einer Trennwandkolonne mit 65 theoretischen Trennstufen zugeführt, wobei im Vergleichsbeispiel eine Trennwandkolonne mit üblicher, das heißt nicht bis oben durchgezogener Trennwand und im Beispiel nach der Erfindung eine bis zum oberen Kolonnenende durchgezogene Trennwand eingesetzt wurde. Die Trennstufen wurden jeweils von unten nach oben gezählt. Im Vergleichsbeispiel wurde die Trennwand thermodynamisch optimiert und war zwischen der 37. und 58. theoretischen Trennstufe bei 4 bar, bzw. zwischen der 38. und 60. theoretischen Trennstufe bei 5 bar, angeordnet.

Die maßgeblichen Verfahrensparameter sind in der nachfolgenden Tabelle zusammengestellt:

| | **Vergleich** | **Erfindung** | **Vergleich** | **Erfindung** |
|---|---|---|---|---|
| Kopfdruck [bar] | 4 | 4 | 5 | 5 |
| ErforderlicheVerdampferleistung [kW] | 29851 | 21174 | 24570 | 20576 |
| Erforderliche Gesamtmenge an Lösungsmittel [kg/h] | 542860 | 373110 | 474830 | 366275 |
| Aufteilungsverhältnis des Lösungsmittels | * 1,32 | ** 2,71 | * 1,0 | ** 2,80 |

| | | | | |
|---|---|---|---|---|
| * am oberen Ende der Trennwand ** auf die Lösungsmittelströme S1/S2 in Fig. 1 | | | | |

Die Ergebnisse in der Tabelle zeigen, dass die erforderliche Verdampferleistung bei 4 bar Kolonnendruck für das erfindungsgemäße Verfahren um 30 % unterhalb des Verfahrens in einer klassischen Trennwandkolonne und bei einem Kolonnendruck von 5 bar um 16 % niedriger ist.

Darüber hinaus ist die erforderliche Gesamtmenge an Lösungsmittel für dieselbe Trennaufgabe bei einem Kolonnendruck von 4 bar nach dem erfindungsgemäßen Verfahren um 31 % geringer als in einer klassischen Trennwandkolonne und bei einem Kolonnendruck von 5 bar um 23 %. Entsprechend niedriger sind die Betriebskosten, insbesondere Energiekosten sowie die Investitionskosten aufgrund des geringeren erforderlichen Kolonnenquerschnittes.

Darüber hinaus hat die klassische Trennwandkolonne gegenüber der erfindungsgemäß eingesetzten Kolonne mit durchgezogener Trennwand den zusätzlichen Nachteil, dass sie weniger flexibel gegenüber Veränderungen des Arbeitsdruckes ist: um die gegebene Trennaufgabe mit der oben aufgeführten Verdampferleistung auszuführen war bei einem Druckanstieg von lediglich 1 bar und zwar von 4 auf 5 bar, eine konstruktive Veränderung der klassischen Trennwandkolonne erforderlich, insofern als die Trennwand vertikal um eine Trennstufe verlegt (von der 37. auf die 38. Trennstufe) und um eine Trennstufe in ihrer Länge verändert, die Zuführung des Ausgangsgemisches um fünf Trennstufen nach unten verlegt (von der 12. Trennstufe des Trennwandbereiches auf die 7. Trennstufe des Trennwandbereichs) und der Seitenabzug um eine Trennstufe nach oben verlegt werden musste.

Demgegenüber war für die erfindungsgemäß eingesetzte Trennwandkolonne mit durchgezogener Trennwand bei einer Änderung des Arbeitsdruckes keinerlei derartige Änderung erforderlich.

## Patentansprüche

1. Verfahren zur Auftrennung eines Ausgangsgemisches (A) aus zwei oder mehreren Komponenten durch Extraktivdestillation mit einem selektiven Lösungsmittel (S) in einer Trennwandkolonne (TKW), wobei
- man das Verfahren in einer Trennwandkolonne (TKW) mit einer in Kolonnenlängsrichtung angeordneten Trennwand (TW) durchführt, die bis zum oberen Kolonnenende durchgezogen ist und die das Kolonneninnere in einen ersten Teilbereich (1), einen zweiten Teilbereich (2) und einen unteren gemeinsamen Kolonnenbereich (3) aufteilt,
- das Ausgangsgemisch (A) dem ersten Teilbereich (1) zuführt, aus dem ersten Teilbereich (1) einen ersten Kopfstrom (B) und aus dem zweiten Teilbereich (2) einen zweiten Kopfstrom (C), mit jeweils vorgegebener Spezifikation, abzieht,
- das selektive Lösungsmittel (S) im oberen Bereich des ersten Teilbereichs (1) oder im oberen Bereich des zweiten Teilbereichs (2) aufgibt,
- die Menge des auf den ersten Teilbereich (1) aufgegebenen Lösungsmittelstromes (S1) oder die Menge des auf den zweiten Teilbereich (2) aufgegebenen Lösungsmittelstromes (S2) dergestalt einstellt, dass die jeweils vorgegebenen Spezifikationen für die Kopfströme (B, C) eingehalten werden,
und wobei aus dem unteren gemeinsamen Kolonnenbereich (3) ein Seitenstrom (D) abgezogen und das beladene Lösungsmittel aus dem Kolonnensumpf (SL) in einem Sumpfverdampfer (V) ausgegast und als gereinigter Lösungsmittelstrom (SR) abgezogen und vorzugsweise in die Extraktivdestillation rezykliert wird.

2. Verfahren nach Anspruch 1, **dadurch gekennzeichnet, dass** man lediglich im oberen Bereich des ersten Teilbereichs (1) einen Strom des selektiven Lösungsmittels (S1) aufgibt.

3. Verfahren nach Anspruch 1, **dadurch gekennzeichnet, dass** man lediglich im oberen Bereich des zweiten Teilbereichs (2) einen Strom des selektiven Lösungsmittel (S2) aufgibt.

4. Verfahren nach einem der Ansprüche 1 bis 3, **dadurch gekennzeichnet, dass** man von einer oder mehrerer thermodynamisch geeigneter Trennstufen einen oder mehrere Flüssigkeitsströme oder -teilströme aus der Trennwandkolonne (TKW) abzieht, durch Wärmeintegration mit dem heißen, entgasten Lösungsmittelstrom (SR) teilweise oder vollständig verdampft und der Trennwandkolonne (TKW) erneut zuführt, vorzugsweise auf derselben Stufe, von der der Flüssigkeitsstrom oder -teilstrom abgezogen wurde.

5. Verwendung des Verfahrens nach einem der Ansprüche 1 bis 4 zur Auftrennung von Kohlenwasserstoffgemischen, insbesondere von C4-Schnitten, von C5-Schnitten oder von Aromatengemischen, bevorzugt von Benzol-Toluol-Xylol-Gemischen oder von Gemischen der isomeren Xylole.

## Claims

1. A process for fractionating a starting mixture (A) of two or more components by extractive distillation using a selective solvent (S) in a dividing wall column (TKW), wherein
- the process is carried out in a dividing wall column (TKW) having a dividing wall (TW) which is aligned in the longitudinal direction of the column and extends to the upper end of the column and divides the interior of the column into a first region (1), a second region (2) and a lower combined column region (3),
- the starting mixture (A) is fed into the first region (1), a first top stream (B) is taken off from the first region (1) and a second top stream (C) is taken off from the second region (2), with each of the streams having a prescribed specification,
- the selective solvent (S) is introduced in the upper part of the first region (1) or in the upper part of the second region (2),
- the flow of solvent (S1) into the first region (1) or the flow of solvent (S2) into the second region (2) is set so that each of the prescribed specifications for the top streams (B, C) are met,
and a side stream (D) is taken off from the lower combined column region (3) and the laden solvent from the bottom of the column (SL) is degassed in a bottom vaporizer (V) and taken off as purified solvent stream (SR) and preferably recycled to the extractive distillation.

2. The process according to claim 1, wherein a stream of the selective solvent (S1) is introduced only in the upper part of the first region (1).

3. The process according to claim 1, wherein a stream of the selective solvent (S2) is introduced only in the upper part of the second region (2).

4. The process according to any of claims 1 to 3, wherein one or more liquid streams or substreams is/are taken off from the dividing wall column (TKW) at one or more thermodynamically suitable theoretical plates, partly or completely vaporized by heat transfer from the hot, degassed solvent stream (SR) and returned to the dividing wall column (TKW), preferably at the same theoretical plate from which the liquid stream or substream had been taken off.

5. The use of the process according to any of claims 1 to 4 for fractionating hydrocarbon mixtures, in particular C₄ fractions, C₅ fractions or aromatic mixtures, preferably benzene/toluene/xylene mixtures or mixtures of the isomeric xylenes.

## Revendications

1. Procédé de séparation d'un mélange initial (A) constitué de deux composants ou plus par distillation extractive avec un solvant sélectif (S) dans une colonne à paroi de séparation (TKW), selon lequel
- le procédé est réalisé dans une colonne à paroi de séparation (TKW) munie d'une paroi de séparation (TW) placée dans la direction longitudinale de la colonne, qui est continue jusqu'à l'extrémité supérieure de la colonne et qui partage l'intérieur de la colonne en une première section (1), une deuxième section (2) et une zone de colonne commune inférieure (3),
- le mélange initial (A) est introduit dans la première section (1), un premier courant de tête (B) est soutiré de la première section (1) et un deuxième courant de tête (C) est soutiré de la deuxième section (2), avec à chaque fois une spécification prédéterminée,
- le solvant sélectif (S) est éliminé dans la zone supérieure de la première section (1) ou dans la zone supérieure de la deuxième section (2),
- la quantité du courant de solvant éliminé dans la première section (1) (S1) ou la quantité du courant de solvant éliminé dans la deuxième section (2) (S2) est ajustée de manière à ce que les spécifications prédéterminées pour les courants de tête (B, C) soient respectées à chaque fois,
et selon lequel un courant latéral (D) est soutiré de la zone de colonne commune inférieure (3) et le solvant chargé est dégazé du fond de la colonne (SL) dans un évaporateur de fond (V) et soutiré sous la forme d'un courant de solvant purifié (SR) et de préférence recyclé dans la distillation extractive.

2. Procédé selon la revendication 1, **caractérisé en ce qu'**un courant du solvant sélectif (S1) n'est éliminé que dans la zone supérieure de la première section (1).

3. Procédé selon la revendication 1, **caractérisé en ce qu'**un courant du solvant sélectif (S2) n'est éliminé que dans la zone supérieure de la deuxième section (2).

4. Procédé selon l'une quelconque des revendications 1 à 3, **caractérisé en ce qu'**un ou plusieurs courants ou courants partiels liquides sont soutirés de la colonne à paroi de séparation (TKW) à partir d'une ou de plusieurs étapes de séparation thermodynamiquement appropriées, partiellement ou totalement évaporés par intégration de chaleur avec le courant de solvant dégazé chaud (SR), et réintroduits dans la colonne à paroi de séparation (TKW), de préférence à la même étape que celle à laquelle le courant ou courant partiel liquide avait été soutiré.

5. Utilisation du procédé selon l'une quelconque des revendications 1 à 4 pour la séparation de mélanges d'hydrocarbures, notamment de coupes en C₄, de coupes en C₅ ou de mélanges de composés aromatiques, de préférence de mélanges benzène-toluène-xylène ou de mélanges des xylènes isomères.
